# EUROPÄISCHE PATENTSCHRIFT

(11) **EP 1 093 582 B1**
(45) Veröffentlichungstag und Bekanntmachung des Hinweises auf die Patenterteilung: **11.09.2002**
(21) Anmeldenummer: 99945904.3
(22) Anmeldetag: 06.07.1999
(51) Int. Cl.: G01N 33/28

(54) **VERFAHREN UND VORRICHTUNG ZUR BESTIMMUNG DER BASE NUMBER (BN) EINES SCHMIERÖLS**
METHOD AND DEVICE FOR DETERMINING THE BASE NUMBER (BN) OF A LUBRICATING OIL
PROCEDE ET DISPOSITIF PERMETTANT DE DETERMINER L'INDICE D'ALCALINITE (BN) D'UNE HUILE LUBRIFIANTE

(30) Priorität: 08.07.1998 DE 19830435
(43) Veröffentlichungstag der Anmeldung: 25.04.2001
(73) Patentinhaber: Winkler, Matthias, 25337 Elmshorn (DE)
(72) Erfinder: Winkler, Matthias, 25337 Elmshorn (DE)
(86) Internationale Anmeldenummer: DE9902063
(87) Internationale Veröffentlichungsnummer: WO00003241

(56) Entgegenhaltungen:
- DE-A- 3 625 741
- DE-A- 3 701 348
- DE-C- 4 204 811

## Beschreibung

Die Erfindung betrifft ein Verfahren zur Bestimmung der Base Number (BN) eines Schmieröls nach dem Oberbegriff des Anspruchs 1 sowie eine Vorrichtung zur Durchführung eines solchen Verfahrens nach dem Oberbegriff des Anspruchs 5.

Dieseselmotoren benötigen für einen langdauernden Betrieb Schmieröle zur Verhinderung des Verschleisses sowie zur Kühlung der Zylinderräume. Solche Öle unterliegen jedoch Alterungsprozessen, insbesondere dadurch, daß säurehaltige Verbrennungsrückstände in das Schmieröl gelangen. Moderne Dieselmotorenöle haben daher einen hohen Anteil an alkalischen Additiven, die sicherstellen sollen, daß immer eine gewisse alkalische Reserve des Schmieröls erhalten bleibt, um die säurehaltigen Verbrennungsrückstände zu neutralisieren.

Die Qualität der alkalischen Reserve des Schmieröls sollte kontinuierlich überwacht werden, um Schäden am Motor zu vermeiden.

Zur Feststellung der alkalischen Reserve eines Schmieröls wird allgemein die Bezeichnung Base Number (BN), früher auch Gesamtbasenzahl genannt, verwendet. Die Base Number definiert dabei die Menge an Säure, absolut oder relativ gemessen, die erforderlich ist, um die basischen Inhaltsstoffe, die in einer definierten Menge Schmieröl enthalten sind, zu neutralisieren.

In der Praxis ist es in vielen Fällen nicht erforderlich, die absolute Base Number zu erhalten, sondern es reicht aus, die prozentuale Abweichung der Base Number von derjenigen eines unverbrauchten Schmieröls festzustellen. Eine in der Schiffahrt durchgesetzte Methode besteht darin, eine Probe des zu bewertenden Schmieröls zusammen mit einem Verdünnungsmittel und einem Reagenz in einen Druckbehälter einzusetzen, und zu ermitteln, welcher Druck sich in dem Druckbehälter nach Reaktion der im Schmieröl vorhandenen basischen Inhaltsstoffe mit der als Reagenz in den Druckbehälter eingeführten Säure, insbesondere Phosphorsäure, einstellt.

Ein solches Verfahren ist in der DE 36 25 741 angegeben.

Da bereits die Base Number eines unverbrauchten Schmieröls mit dem vorgesehenen Einsatzzweck und dem jeweiligen Hersteller stark variiert, müssen die bei der Bewertung eines Schmieröls festgestellten Drücke mit einer Nomierungskurve, die üblicherweise in Form von Diagrammen vom Schmierölhersteller zur Verfügung gestellt wird oder empirisch zu ermitteln ist, korreliert werden.

Es hat sich herausgestellt, daß es mit zunehmender Zahl unterschiedlicher Schmieröle zunehmend schwieriger wird, Normierungskurven der verwendeten Schmieröle vorrätig zu halten oder überhaupt zu erhalten. Die Herstellung eigener Diagramme ist sehr zeitaufwendig und erfordert eine Vielzahl von Versuchen. Es kommt hinzu, daß sich die Additive in einem Schmieröl mit der Zeit ändern, so daß ein ständiges Aktualisieren der Diagramme erforderlich ist.

Die bei den bekannten Verfahren sich einstellenden Drücke liegen je nach Schmierölsorte zwischen 50 und 400 mbar. Daraus ergibt sich außerdem, daß bei niedrig legierten Ölen eine hohe Meßungenauigkeit bei zunehmend schlechter reagierenden Additiven auftritt.

Der Erfindung liegt daher die Aufgabe zugrunde, ein Verfahren zur Bestimmung der Base Number (BN) eines Schmieröls anzugeben, das die Handhabung wesentlich erleichtert und die Meßgenauigkeit erhöht, wobei die Einfachheit der Messung weiterhin gewährleistet bleiben soll. Es ist auch Aufgabe der Erfindung, eine entsprechende Vorrichtung anzugeben.

Diese Aufgabe wird durch die in den Ansprüchen 1, 4 und 5 angegebene Erfindung gelöst. Vorteilhafte Weiterbildungen der Erfindung sind in Unteransprüchen angegeben.

Gemäß dem erfindungsgemäßen Verfahren erfolgt die Bewertung eines Schmieröls in zwei Stufen. In einer ersten Stufe wird die Base Number eines unverbrauchten Schmieröls festgestellt und diese wird auf einen festen Betrag, beispielsweise 100%, nomiert. In einer zweiten Stufe wird dann das zu bewertende Schmieröl unter vergleichbaren Apparate- und Volumenbedingungen wie in der ersten Stufe gemessen, so daß die resultierende Base Number sich als ein Prozentsatz der Base Number eines unverbrauchten Schmieröls ergibt. Aus der festgestellten Base Number des zu bewertenden Schmieröls kann dann die entsprechend erforderliche Maßnahme abgeleitet werden, nämlich Aufbereitung oder Ersatz des Schmieröls.

Ein zur Durchführung des Verfahrens verwendbarer Druckbehälter weist einen Kolben auf, der bei der Messung des unverbrauchten Schmieröls soweit verstellt wird, daß sich am Druckmesser ein Standardwert, z. B. 100%, einstellt. Nach dieser ersten Messung wird eine zweite Messung durchgeführt, bei der das Volumen des Druckbehälters durch den Kolben ebenfalls auf die gleiche Einstellung gebracht wird, wie bei der Messung des unverbrauchten Schmieröls.

Die Bewegung des Kolbens erfolgt vorzugsweise mittels einer Gewindestange in der Druckbehälterwandung. Zur Feststellung der Kolbenhöhe bei der Messung in der ersten Stufe weist die Gewindestange vorzugsweise eine Skaleneinteilung auf, die beispielsweise das absolute Volumen anzeigen kann oder auch das Volumen in relativen Einheiten zum Ausgangsvolumen.

In einer anderen Ausführungsform erfolgt zunächst eine Druckmessung des unverbrauchten Schmieröls. Der erhaltene Druck wird dabei einem bestimmten Volumenwert zugeordnet, der bei der Messung des zu bewertenden Schmieröls durch entsprechende Kolbenverstellung eingestellt wird.

Das Druckmeßgerät weist zu diesem Zweck eine Volumenskala und eine Druckskala auf. Der im zweiten Verfahrensschritt erfaßte Wert an der Volumenskala wird verwendet, um bei der Erfassung des zu bewertenden Schmieröls die Kolbenhöhe entsprechend voreinzustellen.

In diesem Fall ist im zweiten Verfahrensschritt keine Volumenänderung erforderlich.

Anstelle zweier Skalen an einem Druckmeßgerät kann die Messung des unverbrauchten Schmieröls auch in einem zweiten Druckbehälter (ohne Volumenverstellmöglichkeit) erfolgen. Der auf der Skala erfaßte Volumenwert (in Relation zum tatsächlich gemessenen Druckwert) läßt sich dann auf den Druckmeßbehälter mit änderbarem Volumen übertragen.

Durch das erfindungsgemäße Verfahren wird unabhängig von der Ölsorte der Druck eines unverbrauchten Schmieröls immer in Bezug zum gleichen Standardwert erfaßt, so daß sich auch für niedrig legierte Öle eine hohe Meßgenauigkeit ergibt. Das Verfahren läßt sich einfach durchführen und erfordert keine Elektronikschaltungen, was sich im rauhen Alltagsbetrieb auf Schiffen bewährt hat.

In einer weiteren Ausgestaltung der Erfindung kann vorgesehen sein, daß der verwendete Druckbehälter bei gleichem Volumen doppelt ausgeführt ist, wobei in jedem der Behälter ein Kolben angeordnet ist und beide Kolben miteinander fest verbunden sind. In einem der Behälter kann dann die Messung des unverbrauchten Schmieröls und im anderen Behälter gleichzeitig die Messung des zu bewertenden Schmieröls erfolgen. Dabei ist jedem Behälter ein eigenes Druckmeßgerät zugeordnet. Bei gleichzeitiger Verstellung der beiden Kolben wird der Meßwert des unverbrauchten Öls bis auf einen Standardwert eingestellt, so daß sich an dem anderen Druckmeßgerät gleichzeitig der Druckwert des zu bewertenden Schmieröls einstellt. Die beiden Druckmeßgeräte können auch zu einer Einheit kombiniert werden, beispielsweise dadurch, daß zwei Zeiger auf einer gemeinsamen Achse angeordnet werden.

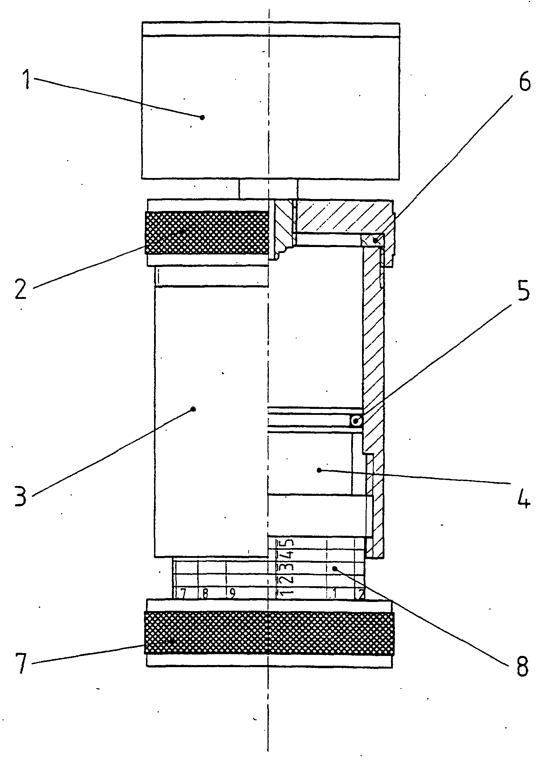

Die Erfindung wird nachstehend anhand eines Ausführungsbeispiels der ersten Ausführungsform näher erläutert. Die einzige Figur zeigt ein Druckmeßgerät mit einem Druckbehälter 3, auf den ein Deckel 2 aufschraubbar ist. Dieser enthält an der Oberseite ein Druckmeßgerät 1. Deckel 2 und Druckbehälter 3 sind über eine Dichtung 6 abgedichtet.

Im unteren Teil des Druckbehälters befindet sich ein Kolben 4, der gegenüber der Innenwandung des Druckbehälters 3 mittels einer Dichtung 5 abgedichtet ist. Der Kolben 4 läßt sich über eine Schraubkappe 7 mittels einer Gewindestange, die in den Boden des Behälters 3 eingesetzt ist, im Behälter 3 auf- und abbewegen. Die Gewindestange ist von einer Skala 8 umgeben, die es erlaubt, die genaue Stellung des Kolbens 4 im Druckbehälter festzustellen.

Das Meßverfahren wird wie folgt durchgeführt:

In den geöffneten Behälter wird eine Probe eines unverbrauchten Schmieröls zusammen mit einem Verdünnungsmittel und einem Reagenz eingebracht. Dabei befindet sich der Kolben 4 in der unteren Stellung (Ausgangsvolumen). Nach Schließen des Deckels entsteht durch die im Inneren des Druckbehälters sich ausbildende Reaktion ein Überdruck, der nach einer gewissen Reaktionszeit einen bestimmten Endwert am Druckmeßgerät 1 anzeigt. Nun wird über die Schraubkappe 7 und die nicht dargestellte Gewindestange der Kolben 4 im Behälter aufwärtsbewegt, so daß der Druck am Druckmeßgerät 1 erhöht wird. Die Schraubkappe 7 wird so lange gedreht, bis der Druck am Druckmeßgerät 1 einen bestimmten Endwert oder Standardwert erreicht hat, beispielsweise 100% oder einen bestimmten absoluten Wert. An der Skala 8 wird der dabei eingestellte Volumenwert festgestellt.

Im nächstfolgenden Schritt wird der Kolben 4 wieder in seine Ausgangsposition zurückgeführt und es wird das zu bewertende Schmieröl anstelle des unverbrauchten Schmieröls in den Meßbehälter zusammen mit dem Reagenz und einem Verdünnungsmittel eingeführt. Nach erneutem Druckaufbau wird der Kolben 4 wieder zu der Position vorgeschoben, bei der sich der Standardwert bei der ersten Messung ergab. Der am Druckmeßgerät angezeigte Druck wird nun festgestellt und zur Bewertung der Base Number (BN) des eingebrachten Schmieröls ausgewertet.

Die Durchführung der Erfindung benötigt daher keine Diagramme mehr, sondern die prüfende Person kann aus dem Vergleich der beiden Messungen unmittelbar auf die Base Number in relativen Einheiten zur Base Number eines unverbrauchten Schmieröls schließen.

Die Messung sowohl des unverbrauchten Schmieröls als auch des zu bewertenden Schmieröls unter identischen Volumenbedingungen ermöglicht den unmittelbaren Vergleich der Öle, ohne daß sich dabei Meßfehler einstellen können. Dabei wird erreicht, daß bei jeder Messung das gleiche Volumen an Luft im Behälter vorhanden ist.

Da die Base Number eines unverbrauchten Schmieröls in der Regel bekannt ist und bei der Messung in der ersten Stufe auf 100% festgelegt wird, kann bei der Feststellung des relativen Prozentualwertes des zu bewertenden Öls durch eine einfache Umrechnung auuch die absolute Base Number des zu bewertenden Öls ermittelt werden

### Bezugszeichenliste

- 1: Druckmeßgerät
- 2: Deckel
- 3: Druckbehälter
- 4: Kolben
- 5: Dichtung
- 6: Dichtung
- 7: Schraubkappe
- 8: Skala

## Patentansprüche

1. Verfahren zur Bestimmung der Base Number (BN) eines Schmieröls mittels eines Druckbehälters, in dem an einem Druckmeßgerät (1) der resultierende Druckanstieg nach Einbringen einer Probe des Schmieröls zusammen mit einem eine Säure enthaltenden Reagenz in den Druckbehälter (3) ermittelt wird, **dadurch gekennzeichnet,**
- **daß** in einem ersten Schritt eine Probe eines unverbrauchten Schmieröls oder eines mit einer bekannten Base Number zusammen mit dem Reagenz in einen Druckbehälter (3) eingebracht wird,
- **daß** nach dem Aufbau des Druckes in einem zweiten Schritt der erhaltene Meßwert des Druckmeßgerätes mit einem gewählten Standardwert in Beziehung gesetzt wird, wobei der Standardwert einem Druck im Druckbehälter bei einem bestimmten reduzierten Volumenwert entspricht,
- **daß** in einem dritten Schritt eine Probe des zu bewertenden Schmieröls zusammen mit dem Reagenz in den entleerten Druckbehälter eingebracht wird, und
- **daß** in einem vierten Schritt das Volumen des Druckbehälters auf den im zweiten Schritt erfaßten Volumenwert reduziert wird, wobei der resultierende Druck am Druckmeßgerät erfaßt und zur Bestimmung der Base Number des eingebrachten Schmieröls ausgewertet wird.

2. Verfahren nach Anspruch 1, **dadurch gekennzeichnet, daß** das Druckmeßgerät (1) eine Volumenskala enthält, die Volumenwerten zugeordnet ist, und daß im vierten Schritt das Volumen des Druckbehälters auf den an der Volumenskala im zweiten Schritt erfaßten Volumenwert eingestellt wird.

3. Verfahren nach Anspruch 1, **dadurch gekennzeichnet, daß** im zweiten Schritt das Volumen des Behälters soweit verkleinert wird, daß der Meßwert des Druckmeßgeräts (1) mit dem gewählten Standardwert übereinstimmt, wobei der eingestellte Volumenwert erfaßt wird, und daß das Volumen des Druckbehälters danach wieder auf das Ausgangsvolumen vergrößert wird.

4. Verfahren nach dem Oberbegriff des Anspruchs 1, **dadurch gekennzeichnet,**
- **daß** in einen ersten Druckbehälter eine Probe eines unverbrauchten Schmieröls oder eines mit einer bekannten Base Number eingebracht wird,
- **daß** in einen zweiten Druckbehälter eine Probe eines zu bewertenden Schmieröls eingebracht wird,
- **daß** nach dem Aufbau der jeweiligen Drücke in den beiden Behältern das Volumen der Behälter im gleichen Maße soweit verkleinert wird, bis der Meßwert des Druckmeßgerätes des ersten Druckbehälters mit einem gewünschten Standardwert übereinstimmt, und
- **daß** der sich entsprechend im zweiten Druckbehälter einstellende Druckwert erfaßt und zur Bestimmung der Base Number des eingebrachten verbrauchten Schmieröls ausgewertet wird.

5. Vorrichtung zur Bestimmung der Base Number (BN) eines Schmieröls zur Durchführung eines Verfahrens nach Anspruch 1, mit einem verschließbaren Druckbehälter, in dem ein Druckmeßgerät (1) zur Feststellung des Innendruckes des Druckbehälters angeordnet ist, **dadurch gekennzeichnet, daß** der Druckbehälter (3) einen beweglichen Kolben (4) zur Volumenänderung des Druckbehälters aufweist.

6. Vorrichtung nach Anspruch 5, **dadurch gekennzeichnet, daß** die Bewegung des Kolbens mittels einer in der Druckbehälterwandung geführten drehbaren Gewindestange erfolgt.

7. Vorrichtung nach Anspruch 5 oder 6, **dadurch gekennzeichnet, daß** eine Anzeigevorrichtung zur Bestimmung des relativen Volumens des Druckbehälters bezüglich des Anfangsvolumens vorgesehen ist.

8. Vorrichtung nach Anspruch 5, **dadurch gekennzeichnet, daß** ein zweiter Druckbehälter vorgesehen ist, der einen zweiten beweglichen Kolben zur Volumenänderung des zweiten Druckbehälters aufweist, wobei die Kolben der Druckbehälter so miteinander verbunden sind, daß bei Verschiebung der Kolben die Volumina der Druckbehälter sich im gleichen Maße verändern, und daß jedem Druckbehälter ein Druckmeßgerät zugeordnet ist.

9. Vorrichtung nach Anspruch 5, **dadurch gekennzeichnet, daß** das Druckmeßgerät eine erste Skala mit einer Volumeneinteilung und eine zweite Skala mit einer Druckeinteilung enthält.

10. Vorrichtung nach Anspruch 7 und Anspruch 9, **dadurch gekennzeichnet, daß** die Skala mit Volumeneinteilung der Anzeigevorrichtung zur Bestimmung des relativen Volumens zugeordnet ist.

## Claims

1. Method of determining the base number (BN) of a lubricating oil by means of a pressure vessel, in which after introducing a sample of the lubricating oil together with an acid-containing reagent into the pressure vessel (3) the resulting pressure rise is determined at a pressure gauge (1), **characterized in**
- **that** in a first step a sample of an unused lubricating oil or of a lubricating oil with a known base number is introduced together with the reagent into a pressure vessel (3),
- **that** after the build-up of the pressure in a second step the obtained measured value of the pressure gauge is related to a selected standard value, wherein the standard value corresponds to a pressure in the pressure vessel for a specific reduced volume value,
- **that** in a third step a sample of the lubricating oil to be assessed is introduced together with the reagent into the emptied pressure vessel, and
- **that** in a fourth step the volume of the pressure vessel is reduced to the volume value acquired in the second step, wherein the resulting pressure is acquired at the pressure gauge and evaluated to determine the base number of the introduced lubricating oil.

2. Method according to claim 1, **characterized in that** the pressure gauge (1) comprises a volume scale, which is associated with volume values, and that in the fourth step the volume of the pressure vessel is adjusted to the volume value acquired at the volume scale in the second step.

3. Method according to claim 1, **characterized in that** in the second step the volume of the vessel is reduced to such an extent that the measured value of the pressure gauge (1) coincides with the selected standard value, wherein the adjusted volume value is acquired, and that the volume of the pressure vessel is then increased back up to the initial volume.

4. Method according to the preamble of claim 1, **characterized in**
- **that** a sample of an unused lubricating oil or of a lubricating oil with a known base number is introduced into a first pressure vessel,
- **that** a sample of a lubricating oil to be assessed is introduced into a second pressure vessel,
- **that** after the build-up of the respective pressures in the two vessels the volume of the vessels is reduced to the same extent until the measured value of the pressure gauge of the first pressure vessel is identical to a desired standard value, and
- **that** the pressure value accordingly arising in the second pressure vessel is acquired and evaluated to determine the base number of the introduced used lubricating oil.

5. Apparatus for determining the base number (BN) of a lubricating oil for effecting a method according to claim 1, comprising a sealable pressure vessel, in which is disposed a pressure gauge (1) for determining the internal pressure of the pressure vessel, **characterized in that** the pressure vessel (3) has a movable piston (4) for varying the volume of the pressure vessel.

6. Apparatus according to claim 5, **characterized in that** the movement of the piston is effected by means of a rotatable threaded rod guided in the pressure vessel wall.

7. Apparatus according to claim 5 or 6, **characterized in that** an indicating apparatus is provided for determining the relative volume of the pressure vessel relative to the initial volume.

8. Apparatus according to claim 5, **characterized in that** a second pressure vessel is provided, which has a second movable piston for varying the volume of the second pressure vessel, wherein the pistons of the pressure vessels are connected to one another in such a way that upon displacement of the pistons the volumes of the pressure vessels vary to the same extent, and that a pressure gauge is associated with each pressure vessel.

9. Apparatus according to claim 5, **characterized in that** the pressure gauge comprises a first scale with a volume graduation and a second scale with a pressure graduation.

10. Apparatus according to claim 7 and claim 9, **characterized in that** the scale with volume graduation is associated with the indicating apparatus for determining the relative volume.

## Revendications

1. Procédé pour définir l'indice de basicité (BN) d'une huile lubrifiante à l'aide d'un récipient sous pression, selon lequel on détermine au niveau d'un manomètre (1) l'augmentation de pression résultante, après avoir placé dans le récipient sous pression (3) un échantillon de l'huile lubrifiante avec un réactif contenant un acide,
**caractérisé**
- **en ce que** lors d'une première phase, on place dans un récipient sous pression (3) un échantillon d'une huile lubrifiante non utilisée ou présentant un indice de basicité connu, avec le réactif,
- **en ce que** lors d'une deuxième phase, après la montée en pression, on met en relation la valeur de mesure obtenue du manomètre avec une valeur standard choisie, cette valeur standard correspondant à une pression dans le récipient sous pression en présence d'une valeur de volume réduite définie,
- **en ce que** lors d'une troisième phase, on place dans le récipient sous pression vidé un échantillon de l'huile lubrifiante à évaluer, avec le réactif, et
- **en ce que** lors d'une quatrième phase, on réduit le volume du récipient sous pression à la valeur de volume enregistrée lors de la deuxième phase, la pression résultante étant enregistrée au niveau du manomètre et étant évaluée pour définir l'indice de basicité de l'huile lubrifiante mise en place.

2. Procédé selon la revendication 1, **caractérisé en ce que** le manomètre (1) contient une échelle de volume qui est associée à des valeurs de volume, et **en ce que** lors de la quatrième phase, on règle le volume du récipient sous pression sur la valeur de volume enregistrée sur ladite échelle de volume lors de la deuxième phase.

3. Procédé selon la revendication 1, **caractérisé en ce que** lors de la deuxième phase, on réduit le volume du récipient jusqu'à ce que la valeur de mesure du manomètre (1) coïncide avec la valeur standard choisie, la valeur de volume réglée étant enregistrée, et **en ce qu'**on augmente ensuite à nouveau le volume du récipient sous pression jusqu'au volume de départ.

4. Procédé selon le préambule de la revendication 1, **caractérisé**
- **en ce qu'**on place dans un premier récipient sous pression un échantillon d'une huile lubrifiante non utilisée ou présentant un indice de basicité connu,
- **en ce qu'**on place dans un second récipient sous pression un échantillon d'une huile lubrifiante à évaluer,
- **en ce que**, après la montée en pression dans les deux récipients, on réduit le volume des récipients de la même manière, jusqu'à ce que la valeur de mesure du manomètre du premier récipient sous pression coïncide avec une valeur standard souhaitée, et
- **en ce que** la valeur de pression qui apparaît en conséquence dans le second récipient sous pression est enregistrée et est évaluée pour définir l'indice de basicité de l'huile lubrifiante utilisée mise en place.

5. Dispositif pour définir l'indice de basicité (BN) d'une huile lubrifiante pour mettre en oeuvre un procédé selon la revendication 1, avec un récipient sous pression apte à être fermé dans lequel est disposé un manomètre (1) pour déterminer la pression interne du récipient sous pression, **caractérisé en ce que** le récipient sous pression (3) comporte un piston mobile (4) destiné à modifier son volume.

6. Dispositif selon la revendication 5, **caractérisé en ce que** le déplacement du piston se fait grâce à une tige filetée rotative guidée dans la paroi du récipient sous pression.

7. Dispositif selon la revendication 5 ou 6, **caractérisé en ce qu'**il est prévu un dispositif d'affichage pour définir le volume relatif du récipient sous pression par rapport au volume initial.

8. Dispositif selon la revendication 5, **caractérisé en ce qu'**il est prévu un second récipient sous pression qui comporte un second piston mobile destiné à modifier son volume, les pistons des récipients sous pression étant reliés entre eux de telle sorte que lors de leur déplacement, les volumes des récipients sous pression varient de la même manière, et **en ce qu'**un manomètre est associé à chaque récipient sous pression.

9. Dispositif selon la revendication 5, **caractérisé en ce que** le manomètre contient une première échelle avec une graduation de volume, et une seconde échelle avec une graduation de pression.

10. Dispositif selon les revendications 7 et 9, **caractérisé en ce que** l'échelle avec la graduation de volume est associée au dispositif d'affichage pour définir le volume relatif.
